# EUROPEAN PATENT SPECIFICATION

(11) **EP 3 054 869 B1**
(45) Date of publication and mention of the grant of the patent: **14.08.2019**
(21) Application number: 14822034.6
(22) Date of filing: 17.12.2014
(51) Int. Cl.: A61B 17/42, A61B 1/303, A61B 8/12

(54) **INTEGRATED UTERINE MANIPULATOR AND SENSOR**
INTEGRIERTER UTERUSMANIPULATOR UND SENSOR
MANIPULATEUR ET CAPTEUR UTÉRINS INTÉGRÉS

(30) Priority: 17.01.2014 US 201461928569 P
(43) Date of publication of application: 17.08.2016
(73) Proprietor: Gyrus ACMI, Inc., d.b.a. Olympus Surgical Technologies America, Southborough, MA 01772 (US)
(72) Inventor: MURDESHWAR, Nikhil, M., Maple Grove, MN 55311 (US); DOBBS, Tracey, L., Delano, MN 55328 (US); LIM, Jyue, Boon, New Brighton, MN 55112 (US); BATCHELOR, Kester, J., Mound, MN 55364 (US); HANNEMAN, John, C., Shakopee, MN 55379 (US)
(74) Representative: Manitz Finsterwald Patent- und Rechtsanwaltspartnerschaft mbB
(86) International application number: PCT/US2014/070898
(87) International publication number: WO 2015/108655

(56) References cited:
- WO-A1-00/32263
- WO-A1-2011/033333
- WO-A2-2010/036721
- US-A1- 2010 145 132
- US-A1- 2012 022 314

## Description

### FIELD

The present disclosure relates to a uterine manipulator, and more particularly, to a uterine manipulator for maneuvering, manipulating and/or clamping the uterus of a patient.

### BACKGROUND

The statements in this section merely provide background information related to the present disclosure and may or may not constitute prior art.

Laparoscopy hysterectomy and myomectomy are common surgical procedures treatment procedures for fibroids. However, with an increasing interest in uterus preservation, treatments are being used on fibroids without excising the uterus. Some examples of treatments used on fibroids include uterine artery embolization (UAE), magnetic resonance image-guided focused ultrasound (MrlgFUS), and radio frequency (RF) ablation. In treatments through which the uterus is preserved, the ablated fibroid is typically left in the uterus to be reabsorbed by the body.

Prior art manipulators are disclosed in WO2010/036721, WO00/32263, US2010/0145132 A1, WO 2011/033333 and US 2012/0022314A1.

Devices used in these procedures include cutting forceps, grasping, coagulation and excision devices. In addition, uterine manipulators are used to help position the uterus for treatment. For example, uterine manipulators are used during surgical procedures to maneuver the uterus and/or cervix. An abdominal ultrasound transducer may be placed on the patient's abdomen outside of the body to assist with viewing the surgical site, however, other body parts may obstruct the viewing area. However, there exists a need for a more accurate way to view the surgical site without creating trauma on the patient.

### SUMMARY

The present disclosure provides a device and method for positioning the uterus for treatment while simultaneously viewing the area near the surgical site, for example, within the uterus, without creating excess trauma on the patient.

In one aspect, which may be combined with or separate from other aspects disclosed herein, a uterine manipulator for manipulation of a uterus of a patient is provided. The uterine manipulator includes an elongate member having a distal end as well as a proximal end, and the elongate member is configured to be inserted into a patient's anatomy. The elongate member defines a center line through a center of the elongate member. An expandable membrane is coupled with the distal end of the elongate member. The expandable membrane has an expanded configuration and a collapsed configuration, wherein in the expanded configuration, the expandable membrane is configured to manipulate the patient's anatomy. A sensor is disposed adjacent to the elongate member and arranged non-coaxially with the center line of the elongate member, wherein the sensor is disposed on a plug that is disposed radially outwardly from the elongate member and the center line, wherein the plug is coupled to the elongate member at the proximal end of the elongate member.

The description also relates to a method of performing transcutaneous abdominal surgery is provided. The method includes inserting a uterine manipulator into a patient's anatomy through a vagina of the patient. The method also includes inserting an ultrasound transducer into the patient's anatomy through the vagina of the patient. Further, the method includes inserting a probe into a patient's anatomy for performing a therapy procedure on the patient. The method also includes activating the probe to perform the therapy procedure.

Accordingly there is contemplated a uterine manipulator for manipulation of a uterus of a patient, the uterine manipulator comprising one or more of the following: an elongate member having a distal end, the elongate member configured to be inserted into a patient's anatomy, the elongate member defining a center line through a center of the elongate member; an expandable membrane coupled with the distal end of the shaft, the expandable membrane having an expanded configuration and a collapsed configuration, wherein in the expanded configuration, the expandable membrane is configured to manipulate the patient's anatomy; and a sensor disposed adjacent to the elongate member and arranged non-coaxially with the central shaft of the elongate member.

Accordingly, there is contemplated a method of performing transcutaneous abdominal surgery, the method comprising one or more of the following steps: inserting a uterine manipulator into a patient's anatomy through a vagina of the patient; inserting an ultrasound transducer into the patient's anatomy through the vagina of the patient; inserting a probe into a patient's anatomy for performing a therapy procedure on the patient; and activating the probe to perform the therapy procedure.

The invention may be further characterized by one or any combination of the features described herein, such as: the sensor is an ultrasound transducer; the ultrasound transducer is disposed radially outwardly from the elongate member and the center line; the expandable membrane is an inflatable balloon; the expandable membrane is configured to manipulate the patient's anatomy by preventing the expandable membrane from exiting the patient's anatomy in the expanded configuration; the ultrasound transducer is configured to move with respect to the elongate member; the ultrasound transducer is rotatable about the elongate member; the uterine manipulator further comprises a sleeve disposed at least partially circumferentially around at least one of the ultrasound transducer and the elongate member; the ultrasound transducer is movable axially along the elongate member; the ultrasound transducer is movable in a radial direction toward and away from the center line; the ultrasound transducer is pivotally attached to the elongate member; the ultrasound transducer is pivotally attached to a sleeve disposed at least partially circumferentially around the elongate member; the ultrasound transducer is fixedly attached to one of the elongate member and a plug fixedly attached to the elongate member; the ultrasound transducer is moveable axially along the elongate member; the ultrasound transducer is movable in a radial direction toward and away from the center line; the ultrasound transducer is selectively fixable to the elongate member; the uterine manipulator further comprises a cervical cup; the ultrasound transducer is attached to the cervical cup; the cervical cup is rotatable about the elongate member; the cervical cup is movable axially along the elongate member; the method further comprises imaging with the ultrasound transducer while simultaneously performing the step of activating the probe to perform the therapy procedure; the method further comprises providing the ultrasound transducer as being disposed adjacent to the uterine manipulator and arranged non-coaxially with the uterine manipulator; and the method further comprises providing the uterine manipulator as having an elongate member and an expandable membrane attached to a distal end of the elongate member.

Further aspects, advantages and areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.
Fig. 1 is a schematic side view of an example of a uterine manipulator, in accordance with the invention;
Fig. 2 is a schematic side view of another example of a uterine manipulator, according to the principles of the present disclosure;
Fig. 3 is a schematic side view of yet another example of a uterine manipulator, in accordance with the principles of the present disclosure;
Fig. 4A is a schematic side view of still another example of a uterine manipulator with a portion cut-away to show a partial cross-sectional view, according to the principles of the present disclosure;
Fig. 4B is a cross-sectional view of the uterine manipulator of Fig. 4A, taken along the line 4B-4B in Fig. 4A, in accordance with the principles of the present disclosure;
Fig. 5 is a schematic side view of still another example of a uterine manipulator, according to the principles of the present disclosure;
Fig. 6A is a schematic side view of still another example of a uterine manipulator in a first position, in accordance with the principles of the present disclosure;
Fig. 6B is a schematic side view of the uterine manipulator of Fig. 6A in a second position, according to the principles of the present disclosure;
Fig. 7 is a side cross-sectional view of still another example of a uterine manipulator, in accordance with the principles of the present disclosure; and
Fig. 8 is a block diagram illustrating a method of performing transcutaneous abdominal surgery, according to the principles of the present disclosure.

### DETAILED DESCRIPTION

The following description is merely exemplary in nature and is not intended to limit the present disclosure, application, or uses.

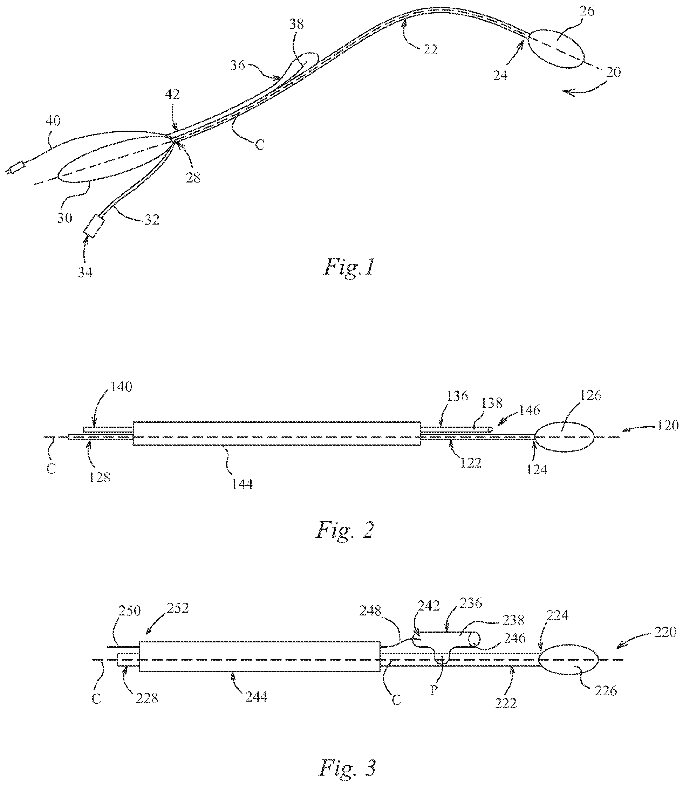

With reference to the figures, wherein like numerals indicate like components, and specifically with reference to Fig. 1, an example of a uterine manipulator in accordance with the invention is illustrated and generally designated at 20. The uterine manipulator 20 is intended to be inserted into a patient's uterus for maneuvering the uterus and/or cervix during various medical examinations and procedures, such as hysterectomies, oophorectomies, fertility studies, removal of tissue specimens, tumor removal, fibroid ablation, among other surgeries, by way of example. The uterine manipulator 20 may be inserted into the uterus through the vagina. The uterine manipulator 20 is used to manipulate, maneuver, position, hold, and/or visualize the uterus and/or the cervix during a surgery.

The uterine manipulator 20 has an elongate member, such as a shaft 22, which may be flexible or semi-flexible, or rigid in some applications. A distal end 24 of the shaft 22 is coupled with an expandable membrane, which is schematically illustrated as an inflatable balloon 26. The shaft 22 is configured to be inserted into a patient's anatomy, and the shaft 22 defines a center line C through a center of the shaft 22. As the shaft 22 is moved, the shaft 22 may curve, and the center line C tracks along the center of the shaft 22. A handle 30 is connected to the proximal end 28 of the shaft 22.

The inflatable balloon 26 has an expanded configuration and a collapsed configuration. The balloon 26 may be inflated and deflated to move between the expanded and collapsed configurations. In the expanded configuration, the balloon 26 is configured to manipulate the patient's anatomy by forming a press-fit with a portion of the patient's uterus, for example, and thus attaching the uterine manipulator 20 to the uterus. Once the uterine manipulator 20 is attached to the uterus, the physician or operator can then move the handle 30 to move and/or contort the uterus to a favorable position for purposes of visualizing or performing a therapy treatment. A passage line 32 having an open end 34 is in fluid communication with the inside of the balloon 26 for inflating/deflating the balloon. The passage line 32 may enter the shaft 22 at the proximal end 28 of the shaft 22.

A sensor 36, such as an ultrasound transducer, is disposed adjacent to the shaft 22 and arranged non-coaxially with the center line C of the shaft 22. The sensor 36 may be an ultrasound transducer for sonographic imaging in the patient's anatomy, such as in the patient's uterus. The sensor 36 is disposed on a plug 38 that is disposed radially outwardly from the shaft 22 and the center line C, in this example. In the example shown in Fig. 1, the plug 38 is coupled to the shaft 22 at the proximal end 28 of the shaft 22. A bundle of lead lines 40 extend from a proximal end 42 of the plug and the proximal end 28 of the shaft 22 to provide power and/or data lines to the sensor 36.

The sensor 36 may be fixedly attached to the shaft 22, or the sensor 36 may be movable with respect to the shaft 22. Figs. 2-7 illustrate certain examples of an articulatable sensor 36, wherein the sensor 36 is movable in one or more directions. These examples should be considered illustrative and not as limiting the invention.

Thus, referring now to Fig. 2, an example of a uterine manipulator is illustrated and generally designated at 120. Like the uterine manipulator 20 of Fig. 1, the uterine manipulator 120 illustrated in Fig. 2 has an elongate member, such as a shaft 122, which may be flexible or semi-flexible, or rigid in some applications. A distal end 124 of the shaft 122 is coupled with an expandable membrane, which is schematically illustrated as an inflatable balloon 126 which may be the same as the inflatable balloon 22 illustrated in Fig. 1, with a passage line such as the passage line 32. The shaft 122 is configured to be inserted into a patient's anatomy, and the shaft 122 defines a center line C through a center of the shaft 122. It should be understood that the proximal end 128 of the shaft 122 is connected to a handle (not shown), which may be similar or the same as the handle 30 illustrated in Fig. 1.

A sensor 136 is disposed adjacent to the shaft 122 and arranged non-coaxially with the center line C of the shaft 122. The sensor 136 may be an ultrasound transducer for sonographic imaging in the patient's anatomy, such as in the patient's uterus. The sensor 136 is disposed on a plug 138 that is disposed radially outwardly from the center line C of the shaft 122, in this example. It should be understood that, while not shown, one or more lead lines extend from a proximal end 142 of the plug 138 to provide power and/or data lines to the sensor 136.

A sleeve 144 surrounds the shaft 122 and the plug 138. The sleeve 144 is disposed at least partially circumferentially around the shaft 122 and the plug 138. One or both of the shaft 122 and the plug 138 are movable along the longitudinal axis of the sleeve 144, which is parallel or coaxial with the center line C of the shaft 122. The sleeve 144 is preferably snugly fit around the plug 138 and the shaft 122, but not so snug that the plug 138 cannot be moved with the respect to the shaft 122. Accordingly, one or both of the plug 138 and the shaft 122 may be slid in the longitudinal direction, to move the distal end 146 of the plug 138 toward and away from the balloon 126 in an axial direction.

Referring now to Fig. 3, another example of a uterine manipulator is illustrated and generally designated at 220. Like the uterine manipulators 20, 120 of Figs. 1 and 2, the uterine manipulator 220 illustrated in Fig. 3 has an elongate member, such as a shaft 222, which may be flexible or semi-flexible, or rigid in some applications. A distal end 224 of the shaft 222 is coupled with an expandable membrane, which is schematically illustrated as an inflatable balloon 226 which may be the same as the inflatable balloon 22 illustrated in Fig. 1, with a passage line such as the passage line 32. The shaft 222 is configured to be inserted into a patient's anatomy, and the shaft 222 defines a center line C through a center of the shaft 222. It should be understood that the proximal end 228 of the shaft 222 is connected to a handle (not shown), which may be similar to or the same as the handle 30 illustrated in Fig. 1.

A sensor 236 is disposed adjacent to the shaft 222 and arranged non-coaxially with the center line C of the shaft 222. The sensor 236 may be an ultrasound transducer for sonographic imaging in the patient's anatomy, such as in the patient's uterus. The sensor 236 is disposed on a plug 238 that is disposed radially outwardly from the shaft 222 and the center line C of the shaft 222, in this example. The plug 238 is pivotally attached to the shaft 222 through a pivot joint, such as a pivot pin P. Thus, the plug 238 is fixedly attached to the shaft 322, in this variation. A cable 248 is attached to a proximal end 242 of the plug 238. One or more lead lines for the sensor 236 may extend through the cable 248.

A sleeve 244 surrounds the shaft 222 and the cable 248. The sleeve 244 is disposed at least partially circumferentially around the shaft 222 and the cable 248. The shaft 222 may be movable along the longitudinal axis of the sleeve 244, which is parallel or coaxial with the center line C of the shaft 222.

The cable 248 extends from the proximal end 242 of the plug 238 and through the lumen of the sleeve 244. When a proximal end 250 of the cable 248 is pushed or pulled at the proximal end 252 of the sleeve 244, the plug 238 and the sensor 236 rotate in a radial direction with respect to the shaft 222 and the center line C about the pivot joint or pin P. Accordingly, the plug 238 is movable in a radial direction toward and away from the center line C. The distal end 246 of the plug 238, which may include the sensor 236, moves away from the center line C when the cable 248 is pulled.

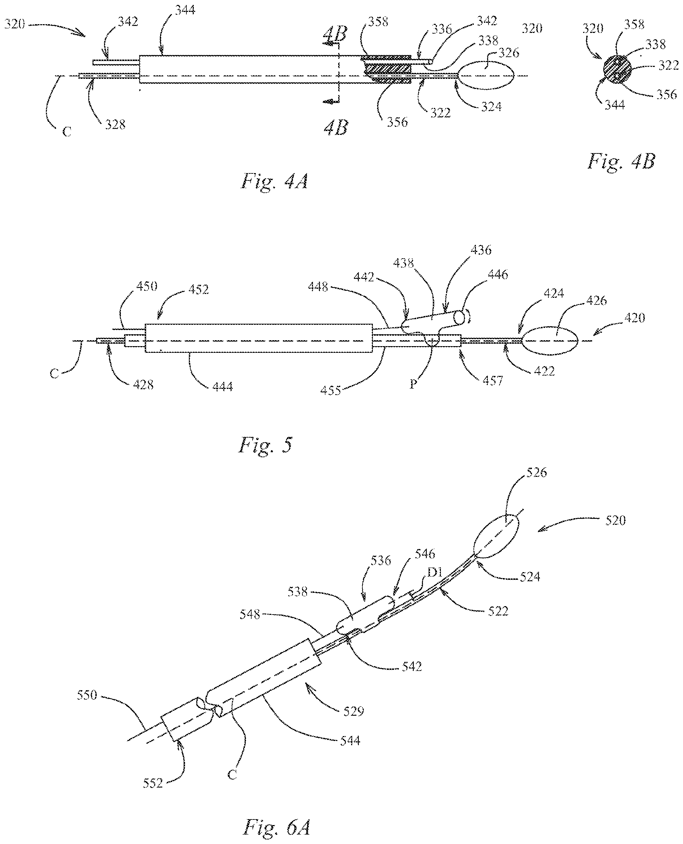

Referring now to Figs. 4A and 4B, yet another example of a uterine manipulator is illustrated and generally designated at 320. Like the uterine manipulators 20, 120, 220 of Figs. 1-3, the uterine manipulator 320 illustrated in Figs. 4A and 4B has an elongate member, such as a shaft 322, which may be flexible or semi-flexible, or rigid in some applications. A distal end 324 of the shaft 322 is coupled with an expandable membrane, which is schematically illustrated as an inflatable balloon 326 which may be the same as the inflatable balloon 22 illustrated in Fig. 1, with a passage line such as the passage line 32. The shaft 322 is configured to be inserted into a patient's anatomy, and the shaft 322 defines a center line C through a center of the shaft 322. It should be understood that the proximal end 328 of the shaft 322 is connected to a handle (not shown), which may be similar to or the same as the handle 30 illustrated in Fig. 1.

A sensor 336 is disposed adjacent to the shaft 322 and arranged non-coaxially with the center line C of the shaft 322. The sensor 336 may be an ultrasound transducer for sonographic imaging in the patient's anatomy, such as in the patient's uterus. The sensor 336 is disposed on a plug 338 that is disposed radially outwardly from the shaft 322 and the center line C, in this example. It should be understood that, while not shown, one or more lead lines extend from a proximal end 342 of the plug 338 to provide power and/or data lines to the sensor 336.

A sleeve 344 surrounds the shaft 322 and the plug 338. The sleeve 344 is disposed at least partially circumferentially around the shaft 322 and the plug 338. One or both of the shaft 322 and the plug 338 are movable along the longitudinal axis of the sleeve 344, which is parallel or coaxial with the center line C of the shaft 322. One or both of the plug 338 and the shaft 322 may be slid in the longitudinal direction, to move the distal end 346 of the plug 338 toward and away from the balloon 326 in an axial direction.

In the illustrated variation, the sleeve 344 forms a first lumen 354 and a second lumen 356 therein, extending through the sleeve 344. As such, the shaft 322 and the plug 338 are rotatable with respect to each other. For example, the sleeve 344 may be slid in a rotational direction around the center line C of the shaft 322, so that the plug 338 may be rotated around the shaft 322. Accordingly, in the variation of Figs. 4A-4B, the sensor 336 is movable in both an axial direction and a rotational direction with respect to the shaft 322.

Referring now to Fig. 5, still another example of a uterine manipulator is illustrated and generally designated at 420. Like the uterine manipulators 20, 120, 220, 320 of Figs. 1-4B, the uterine manipulator 420 illustrated in Fig. 5 has an elongate member, such as a shaft 422, which may be flexible or semi-flexible, or rigid in some applications. A distal end 424 of the shaft 422 is coupled with an expandable membrane, which is schematically illustrated as an inflatable balloon 426 which may be the same as the inflatable balloon 22 illustrated in Fig. 1, with a passage line such as the passage line 32. The shaft 422 is configured to be inserted into a patient's anatomy, and the shaft 422 defines a center line C through a center of the shaft 422. It should be understood that the proximal end 428 of the shaft 422 is connected to a handle (not shown), which may be the same as or similar to the handle 30 illustrated in Fig. 1.

An inner sleeve 455 surrounds the shaft 422, such that the shaft 422 is axially movable within the inner sleeve 455 to slide the shaft 422 within the lumen of the inner sleeve 455. The inner sleeve 455 is disposed at least partially circumferentially around the shaft 422. Accordingly, the shaft 422 may be slid within the inner sleeve 455 to move the inflatable balloon toward and away from the distal end 457 of the inner sleeve 455.

A sensor 436 is disposed adjacent to the shaft 322 and arranged non-coaxially with the center line C of the shaft 422. The sensor 436 may be an ultrasound transducer for sonographic imaging in the patient's anatomy, such as in the patient's uterus. The sensor 436 is disposed on a plug 438 that is disposed radially outwardly from the shaft 422 and the center line C, in this example. The plug 438 is pivotally attached to the inner sleeve 455 through a pivot joint, such as a pivot pin P. Thus, the plug 438 is fixedly attached to the inner sleeve 455, in this variation. A cable 448 is attached to a proximal end 442 of the plug 438. One or more lead lines for the sensor 436 may extend through the cable 448.

An outer sleeve 444 surrounds the shaft 422, the inner sleeve 455, and the cable 448. More particularly, the outer sleeve 444 is disposed at least partially circumferentially around the inner sleeve 455, the shaft 422, and the cable 448. The cable 448 extends from the proximal end 442 of the plug 438 and through the lumen of the outer sleeve 444. The inner sleeve 455 may be movable along the longitudinal axis of the outer sleeve 444, which is parallel or coaxial with the center line C of the shaft 422.

When a proximal end 450 of the cable 448 is pushed or pulled at the proximal end 452 of the outer sleeve 444, the plug 438 and the sensor 436 rotate in a radial direction with respect to the shaft 422 and the center line C. Accordingly, the plug 438 is movable in a radial direction toward and away from the center line C. The distal end 446 of the plug 238, which may include the sensor 436, moves away from the center line C when the cable 448 is pulled.

The inner sleeve 455 and the shaft 422 are rotatable with respect to each other. For example, the inner sleeve 455 may be rotated by sliding the inner sleeve 455 in a rotational direction around the center line C of the shaft 422. Since the plug 438 is rotationally fixed to the inner sleeve 455, the plug 438 (and the sensor 436) may be rotated around the shaft 422.

Accordingly, in the variation of Fig. 5, the sensor 336 is movable in all three of an axial direction, a rotational direction, and a radial direction (toward and away from the center line C) with respect to the shaft 422.

Referring now to Figs. 6A-6B, still another example of a uterine manipulator is illustrated and generally designated at 520. Like the uterine manipulators 20, 120, 220, 320, 420 of Figs. 1-5, the uterine manipulator 520 illustrated in Figs. 6A-6B has an elongate member, such as a shaft 522, which may be flexible or semi-flexible, or having rigid sections, in some applications. A distal end 524 of the shaft 522 is coupled with an expandable membrane, which is schematically illustrated as an inflatable balloon 526 which may be the same as the inflatable balloon 22 illustrated in Fig. 1, with a passage line such as the passage line 32. The shaft 522 is configured to be inserted into a patient's anatomy, and the shaft 522 defines a center line C through a center of the shaft, which extends through a sleeve 544. As with the other shafts 22, 122, 222, 322, 422 described herein, the shaft 522 may curve, and the center line C tracks along the center of the shaft 522 even when the shaft 522 is curved. The proximal end (not shown) of the shaft 522 is connected to a handle (not shown), which may be similar or the same as the handle 30 illustrated in Fig. 1.

A sensor 536 is disposed adjacent to the shaft 522 and arranged non-coaxially with the center line C of the shaft 522. The sensor 536 may be an ultrasound transducer for sonographic imaging in the patient's anatomy, such as in the patient's uterus. The sensor 536 is disposed on a plug 538 that is disposed radially outwardly from the shaft 522 and the center line C, in this example. The plug 538 may be clamped onto the shaft 522 with a press-fit, so that the plug 538 is fixedly attached to the shaft 522, with no pivot pin. The plug 538 may alternatively be slid over the shaft 522 with a slip fit. Thus, the plug 538 is fixedly or slidably attached to the shaft 522, in this variation. A cable 548 is attached to a proximal end 542 of the plug 538. One or more lead lines for the sensor 536 may extend through the cable 548.

The sleeve 544 surrounds the shaft 522 and the cable 548. The sleeve 544 is disposed at least partially circumferentially around the shaft 522 and the cable 548. The shaft 522 may be movable along the longitudinal axis of the sleeve 544, which is parallel to or coaxial with the center line C of the shaft 522 at the proximal portion 529 of the shaft 522.

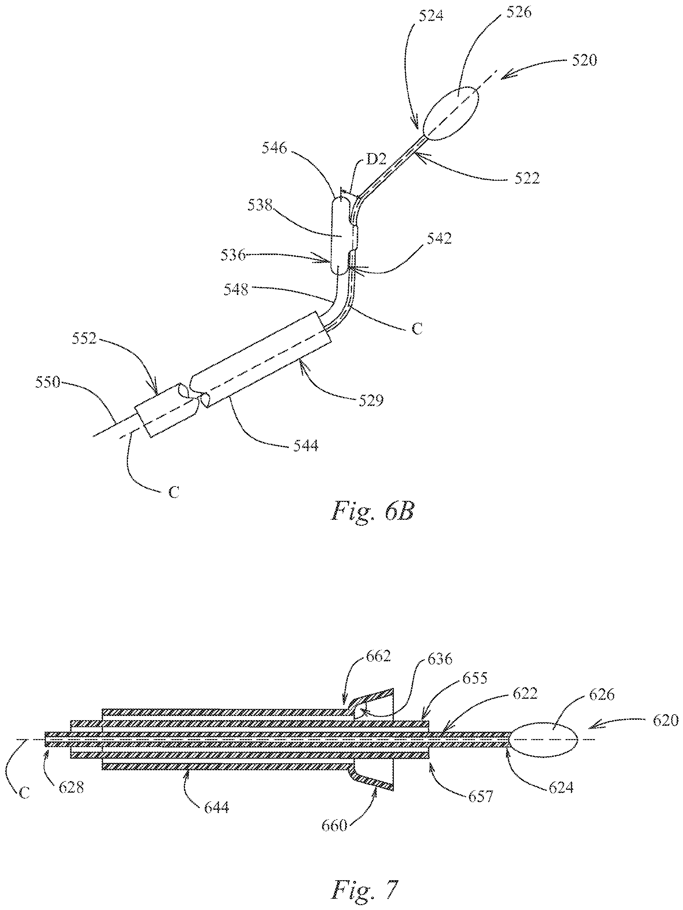

The cable 548 extends from the proximal end 542 of the plug 538 and through the lumen of the sleeve 544. When a proximal end 550 of the cable 548 is pulled at the proximal end 552 of the sleeve 544, the proximal end 542 of the plug 538 is pulled toward the center line C of the shaft 522, causing the distal end 546 of the plug 538 to rotate radially away from the center line C as shown in Fig. 6B. Fig. 5A shows the plug 538 and shaft 522 prior to pulling on the cable 548, and Fig. 5B shows the state of the plug 538 and shaft 522 subsequent to pulling on the proximal end 550 of the cable 548. Accordingly, the shaft 522 can be radially moved without the use of a pivot pin, as illustrated in Fig. 6B. The plug 538 and the sensor 536 rotate in a radial direction with respect to the shaft 522 and the center line C.

Accordingly, the plug 538 is movable in a radial direction toward and away from the center line C. The distal end 546 of the plug 538, which may include the sensor 536, moves away from the center line C when the cable 548 is pulled. Prior to pulling on the cable 548, as shown in Fig. 6A, the distal end 546 of the plug 538 is a distance D1 from the center line C. When the cable 548 is pulled, the distal end 546 moves away from the center line C, and therefore, as shown in Fig. 6B, the distal end 546 is disposed a distance D2 from the center line C. D2 can have various values as the distal end 546 moves farther from the center line C when the cable 548 is pulled, but D2 is greater than D1.

Referring now to Fig. 7, yet another example of a uterine manipulator is illustrated and generally designated at 620. Like the uterine manipulators 20, 120, 220, 320, 420, 520 of Figs. 1-6B, the uterine manipulator 620 illustrated in Fig. 7 has an elongate member, such as a shaft 622, which may be flexible or semi-flexible, or rigid in some applications. A distal end 624 of the shaft 622 is coupled with an expandable membrane, which is schematically illustrated as an inflatable balloon 626 which may be the same as the inflatable balloon 22 illustrated in Fig. 1, with a passage line such as the passage line 32. The shaft 622 is configured to be inserted into a patient's anatomy, and the shaft 622 defines a center line C through a center of the shaft 622. It should be understood that the proximal end 628 of the shaft 622 is connected to a handle (not shown), which may be the same as or similar to the handle 30 illustrated in Fig. 1.

An inner sleeve 655 surrounds the shaft 622, such that the shaft 622 is axially movable within the inner sleeve 655 to slide the shaft 622 within the lumen of the inner sleeve 655. The inner sleeve 655 is disposed at least partially circumferentially around the shaft 622. Accordingly, the shaft 622 may be slid within the inner sleeve 655 to move the inflatable balloon 626 toward and away from the distal end 657 of the inner sleeve 655.

An outer sleeve 644 surrounds the shaft 422 and the inner sleeve 655. More particularly, the outer sleeve 644 is disposed at least partially circumferentially directly around the inner sleeve 655, and also at least partially circumferentially around the shaft 622 that is disposed in the inner sleeve 655. The inner sleeve 655 may be movable along the longitudinal axis of the outer sleeve 644, which is parallel or coaxial with the center line C of the shaft 622.

A cervical cup 660 is formed on or attached to the distal end 662 of the outer sleeve 644. A sensor 636 may be disposed on or within the cervical cup 660 and fixedly attached to the cervical cup 660. The sensor 636 may be an ultrasound transducer for sonographic imaging in the patient's anatomy, such as in the patient's uterus. One or more lead lines (not shown) for the sensor 636 may extend through the outer sleeve 644.

The inner sleeve 655 and the outer sleeve 644 may be rotatable with respect to each other. For example, the outer sleeve 644 may be slid in a rotational direction around the inner sleeve 655, about the center line C of the shaft 622. Since the sensor 636 is rotationally fixed to the outer sleeve 644, the sensor 636 may be rotated around the shaft 622 along with the cervical cup 660 by virtue of sliding the outer sleeve 644 rotationally with respect to the inner sleeve 655. Accordingly, in the variation of Fig. 7, the sensor 636 is movable in an axial direction and a rotational direction with respect to the shaft 622.

In some variations, the sensor 36, 136, 236, 336, 436, 536, 636 (which may include an ultrasound transducer, as described above) may be selectively fixable to the shaft 22, 122, 222, 322, 422, 522, 622. The sensor 36, 136, 236, 336, 436, 536, 636 may be selectively fixable in an axial direction, a rotational direction, and/or a radial direction, if desired. In other words, the sensor 36, 136, 236, 336, 436, 536, 636 may be fixed to the shaft 22, 122, 222, 322, 422, 522, 622 to prevent axial movement therebetween, radial movement therebetween, and/or rotational movement therebetween.

Each component of the uterine manipulator 20, 120, 220, 320, 420, 520, 620 may be formed of a biocompatible material, for example, the shafts 22, 122, 222, 322, 422, 522, 622 and the sleeves 144, 244, 344, 444, 455, 544, 644, 655 may be formed of aluminum, a polymer, or Nitinol, such that the shafts 22, 122, 222, 322, 422, 522, 622 and the sleeves 144, 244, 344, 444, 455, 544, 644, 655 may be twisted or slightly bent to maneuver through the patient's anatomy. In other words, the shafts 22, 122, 222, 322, 422, 522, 622 and the sleeves 144, 244, 344, 444, 455, 544, 644, 655 may be at least partially flexible.

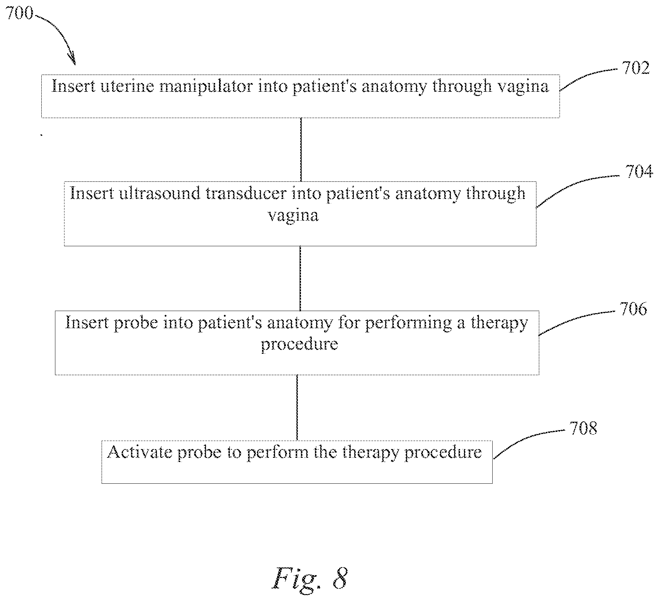

Referring now to Fig. 8, a method of performing transcutaneous abdominal surgery is disclosed and schematically illustrated at 700. The method 700 includes a step 702 of inserting a uterine manipulator into a patient's anatomy through a vagina of the patient. The uterine manipulator may be, but need not necessarily be, one of the uterine manipulators 20, 120, 220, 320, 420, 520, 620 described above.

The method 700 also includes a step 704 of inserting an ultrasound transducer into the patient's anatomy through the vagina of the patient. If one of the uterine manipulators 20, 120, 220, 320, 420, 520, 620 described above is used, the ultrasound transducer will be an integrated part of the uterine manipulator 20, 120, 220, 320, 420, 520, 620 and will necessarily be inserted into the patient's anatomy when the uterine manipulator 20, 120, 220, 320, 420, 520, 620 is inserted into the patient's anatomy.

The method 700 further includes a step 706 of inserting a probe into a patient's anatomy for performing a therapy procedure on the patient. Such a probe may be a probe for performing an RFITT procedure or another instrument designed to ablate a fibroid, by way of example. In one variation, the probe may be a bipolar fibroid ablation needle operable to provide a bipolar surgical tissue necrosis effect.

The method 700 also includes a step 708 of activating the probe to perform the therapy procedure. The step 708 of activating the probe may be performed while the uterine manipulator and the ultrasound transducer are disposed in the patient's anatomy, with the proximal end of the uterine manipulator extending from the patient's vagina, by way of example. Thus, the method 700 may include imaging with the ultrasound transducer while simultaneously performing the step of activating the probe to perform the therapy procedure.

In one variation, the disclosed method may be used in a RFITT fibroid procedure. Such a procedure may include steps of installing a port in the patient's umbilicus and inserting a lap video scope through the port. The uterine manipulator, which is inserted through the vagina in step 702, is then used to manipulate the uterus to present the fibroid for treatment. The endovaginal ultrasound transducer, which is inserted through the vagina in step 704 (may be simultaneous with step 702), is activated adjacent to the uterine manipulator. In step 706, the RFITT probe may be inserted transcutaneously and direct visualization may be used to guide the RFITT probe toward the fibroid. The RFITT probe is positioned inside the fibroid with ultrasound imaging generated from the endovaginal ultrasound transducer inserted in step 704. The treatment end point may also be determined with the ultrasound imaging by the endovaginal ultrasound transducer and/or by direct visualization.

The description of the invention is merely exemplary in nature and variations that do not depart from the gist of the invention are intended to be within the scope of the invention. Such variations are not to be regarded as a departure from the spirit and scope of the invention. For example, variations in the various figures can be combined with each without departing from the spirit and scope of the present disclosure.

For example, it should be understood that the method 700 is merely an example, and variations may occur without departing from the spirit and scope of the invention, as defined by the claims. Likewise, the specific illustrations of the uterine manipulators 20, 120, 220, 320, 420, 520, 620 are examples and are not meant to limit the invention in any way.

The preferred embodiment of the present invention has been disclosed. A person of ordinary skill in the art would realize, however, that certain modifications would come within the teachings of this invention. Therefore, the following claims should be studied to determine the true scope and content of the invention.

Any numerical values recited in the above application include all values from the lower value to the upper value in increments of one unit provided that there is a separation of at least 2 units between any lower value and any higher value. As an example, if it is stated that the amount of a component or a value of a process variable such as, for example, temperature, pressure, time and the like is, for example, from 1 to 90, preferably from 20 to 80, more preferably from 30 to 70, it is intended that values such as 15 to 85, 22 to 68, 43 to 51, 30 to 32 etc. are expressly enumerated in this specification. For values which are less than one, one unit is considered to be 0.0001, 0.001, 0.01 or 0.1 as appropriate. These are only examples of what is specifically intended and all possible combinations of numerical values between the lowest value and the highest value enumerated are to be considered to be expressly stated in this application in a similar manner.

Unless otherwise stated, all ranges include both endpoints and all numbers between the endpoints, the use of "about" or "approximately" in connection with a range apply to both ends of the range. Thus, "about 20 to 30" is intended to cover "about 20 to about 30", inclusive of at least the specified endpoints.

The disclosures of all articles and references, including patent applications and publications, are incorporated by reference for all purposes.

The term "consisting essentially of" to describe a combination shall include the elements, ingredients, components or steps identified, and such other elements ingredients, components or steps that do not materially affect the basic and novel characteristics of the combination.

The use of the terms "comprising" or "including" describing combinations of elements, ingredients, components or steps herein also contemplates embodiments that consist essentially of the elements, ingredients, components or steps.

## Claims

1. A uterine manipulator (20) for manipulation of a uterus of a patient, the uterine manipulator (20) comprising:
an elongate member (22) having a distal end (24) as well as a proximal end (28), the elongate member (22) being configured to be inserted into a patient's anatomy, the elongate member (22) defining a center line (C) through a center of the elongate member (22);
an expandable membrane (26) coupled with the distal end (24) of the elongate member (22), the expandable membrane (26) having an expanded configuration and a collapsed configuration, wherein in the expanded configuration, the expandable membrane (26) is configured to manipulate the patient's anatomy; and a sensor (36) disposed adjacent to the elongate member (22) and arranged non-coaxially with the center line of the elongate member (22), wherein the sensor (36) is disposed on a plug (38) that is disposed radially outwardly from the elongate member (22) and the center line (C), wherein the plug (38) is coupled to the elongate member (22) at the proximal end (28) of the elongate member (22).

2. The uterine manipulator (20) of claim 1, wherein the sensor (26) comprises an ultrasound transducer.

3. The uterine manipulator (20) of claim 2, wherein the ultrasound transducer is disposed radially outwardly from the elongate member (22) and the center line (C).

4. The uterine manipulator (20) of any one of claims 1 through 3, wherein the expandable membrane (26) is an inflatable balloon.

5. The uterine manipulator (20) of any one of claims 1 through 4, wherein the expandable membrane (26) is configured to manipulate the patient's anatomy by preventing the expandable membrane (26) from exiting the patient's anatomy in the expanded configuration.

6. The uterine manipulator (20) of any one of claims 1 through 5, wherein the ultrasound transducer (26) is movable with respect to the elongate member (22).

7. The uterine manipulator (20) of any one of claims 1 through 6, wherein the ultrasound transducer is rotatable about the elongate member (22).

8. The uterine manipulator (20) of any one of claims 1 through 7, further comprising a sleeve disposed at least partially circumferentially around at least one of the ultrasound transducer and the elongate member (22).

9. The uterine manipulator (20) of any one of claims 1 through 6, wherein the ultrasound transducer is movable axially along the elongate member (22).

10. The uterine manipulator (20) of claim 9, further comprising a sleeve disposed at least partially circumferentially around at least one of the ultrasound transducer and the elongate member (22).

11. The uterine manipulator (20) of any one of claims 1 through 6, wherein the ultrasound transducer is movable in a radial direction toward and away from the center line.

12. The uterine manipulator (20) of claim 11, wherein the ultrasound transducer is pivotally attached to one of the elongate member and a sleeve disposed at least partially circumferentially around the elongate member.

13. The uterine manipulator (20) of any one of claims 1 through 6, wherein the ultrasound transducer is fixedly attached to one of the elongate member (22) and the plug () that is fixedly attached to the elongate member.

14. The uterine manipulator (20) of claim 7, wherein the ultrasound transducer is movable axially along the elongate member, and wherein the ultrasound transducer is movable in a radial direction toward and away from the center line.

15. The uterine manipulator (20) of claim 14, wherein the ultrasound transducer is selectively fixable to the elongate member.

16. The uterine manipulator (20) of any one of claims 1 through 6, further comprising a cervical cup, the ultrasound transducer being attached to the cervical cup, the cervical cup being at least one of rotatable about the elongate member and movable axially along the elongate member.

## Patentansprüche

1. Uterusmanipulator (20) zur Manipulation eines Uterus eines Patienten, wobei der Uterusmanipulator (20) umfasst:
ein längliches Element (22) mit einem distalen Ende (24) wie auch einem proximalen Ende (28), wobei das längliche Element (22) zum Einsetzen in die Anatomie eines Patienten konfiguriert ist, wobei das längliche Element (22) eine Mittellinie (C) durch ein Zentrum des länglichen Elements (22) definiert;
eine ausdehnbare Membran (26), die mit dem distalen Ende (24) des länglichen Elements (22) gekoppelt ist, wobei die ausdehnbare Membran (26) eine ausgedehnte Konfiguration und eine zusammengefallene Konfiguration aufweist, wobei in der ausgedehnten Konfiguration die ausdehnbare Membran (26) derart konfiguriert ist, die Anatomie des Patienten zu manipulieren; und
einen Sensor (36), der benachbart dem länglichen Element (22) angeordnet und nicht koaxial mit der Mittellinie des länglichen Elements (22) angeordnet ist, wobei der Sensor (36) an einem Ansatz (38) angeordnet ist, der radial auswärts von dem länglichen Element (22) und der Mittellinie (C) angeordnet ist, wobei der Ansatz (38) mit dem länglichen Element (22) an dem proximalen Ende (28) des länglichen Elements (22) gekoppelt ist.

2. Uterusmanipulator (20) nach Anspruch 1, wobei der Sensor (26) einen Ultraschallwandler umfasst.

3. Uterusmanipulator (20) nach Anspruch 2, wobei der Ultraschallwandler radial auswärts von dem länglichen Element (22) und der Mittellinie (C) angeordnet ist.

4. Uterusmanipulator (20) nach einem der Ansprüche 1 bis 3, wobei die ausdehnbare Membran (26) ein aufblasbarer Ballon ist.

5. Uterusmanipulator (20) nach einem der Ansprüche 1 bis 4, wobei die ausdehnbare Membran (26) derart konfiguriert ist, die Anatomie des Patienten dadurch zu manipulieren, dass verhindert wird, dass die ausdehnbare Membran (26) die Anatomie des Patienten in der ausgedehnten Konfiguration verlässt.

6. Uterusmanipulator (20) nach einem der Ansprüche 1 bis 5, wobei der Ultraschallwandler (26) in Bezug auf das längliche Element (22) bewegbar ist.

7. Uterusmanipulator (20) nach einem der Ansprüche 1 bis 6, wobei der Ultraschallwandler um das längliche Element (22) drehbar ist.

8. Uterusmanipulator (20) nach einem der Ansprüche 1 bis 7, ferner mit einer Hülse, die zumindest teilweise um den Umfang zumindest eines des Ultraschallwandlers und des länglichen Elements (22) angeordnet ist.

9. Uterusmanipulator (20) nach einem der Ansprüche 1 bis 6, wobei der Ultraschallwandler axial entlang des länglichen Elements (22) bewegbar ist.

10. Uterusmanipulator (20) nach Anspruch 9, ferner mit einer Hülse, die zumindest teilweise um den Umfang zumindest eines von dem Ultraschallwandler und dem länglichen Element (22) angeordnet ist.

11. Uterusmanipulator (20) nach einem der Ansprüche 1 bis 6, wobei der Ultraschallwandler in einer radialen Richtung zu und weg von der Mittellinie bewegbar ist.

12. Uterusmanipulator (20) nach Anspruch 11, wobei der Ultraschallwandler schwenkbar an einem von dem länglichen Element und einer Hülse befestigt ist, die zumindest teilweise um den Umfang des länglichen Elements angeordnet ist.

13. Uterusmanipulator (20) nach einem der Ansprüche 1 bis 6, wobei der Ultraschallwandler fest an einem von dem länglichen Element (22) und dem Ansatz (38) befestigt ist, der fest an dem länglichen Element befestigt ist.

14. Uterusmanipulator (20) nach Anspruch 7, wobei der Ultraschallwandler axial entlang des länglichen Elements bewegbar ist, und wobei der Ultraschallwandler in einer radialen Richtung zu und weg von der Mittellinie bewegbar ist.

15. Uterusmanipulator (20) nach Anspruch 14, wobei der Ultraschallwandler selektiv an dem länglichen Element anbringbar ist.

16. Uterusmanipulator (20) nach einem der Ansprüche 1 bis 6, ferner mit einer Zervikalkappe, wobei der Ultraschallwandler an der Zervikalkappe befestigt ist, wobei die Zervikalkappe um das längliche Element drehbar und/oder axial entlang des länglichen Elements bewegbar ist.

## Revendications

1. Manipulateur utérin (20) pour la manipulation d'un utérus d'une patiente, le manipulateur utérin (20) comprenant :
un élément allongé (22) ayant une extrémité distale (24) ainsi qu'une extrémité proximale (28), l'élément allongé (22) étant configuré pour être inséré dans l'anatomie d'une patiente, l'élément allongé (22) définissant une ligne centrale (C) à travers un centre de l'élément allongé (22) ;
une membrane expansible (26) couplée à l'extrémité distale (24) de l'élément allongé (22), la membrane expansible (26) ayant une configuration en expansion et une configuration contractée, dans lequel, dans la configuration en expansion, la membrane expansible (26) est configurée pour manipuler l'anatomie de la patiente ; et
un capteur (36) disposé adjacent à l'élément allongé (22) et agencé de manière non coaxiale avec la ligne centrale de l'élément allongé (22), dans lequel le capteur (36) est disposé sur un plot (38) qui est disposé radialement à l'extérieur de l'élément allongé (22) et de la ligne centrale (C), dans lequel le plot (38) est couplé à l'élément allongé (22) à l'extrémité proximale (28) de l'élément allongé (22).

2. Manipulateur utérin (20) selon la revendication 1, dans lequel le capteur (36) comprend un transducteur à ultrasons.

3. Manipulateur utérin (20) selon la revendication 2, dans lequel le transducteur à ultrasons est disposé radialement à l'extérieur de l'élément allongé (22) et de la ligne centrale (C).

4. Manipulateur utérin (20) selon l'une quelconque des revendications 1 à 3, dans lequel la membrane expansible (26) est un ballon gonflable.

5. Manipulateur utérin (20) selon l'une quelconque des revendications 1 à 4, dans lequel la membrane expansible (26) est configurée pour manipuler l'anatomie de la patiente en empêchant que la membrane expansible (26) sorte de l'anatomie de la patiente dans la configuration en expansion.

6. Manipulateur utérin (20) selon l'une quelconque des revendications 1 à 5, dans lequel le transducteur à ultrasons (36) est déplaçable par rapport à l'élément allongé (22).

7. Manipulateur utérin (20) selon l'une quelconque des revendications 1 à 6, dans lequel le transducteur à ultrasons est capable de rotation autour de l'élément allongé (22).

8. Manipulateur utérin (20) selon l'une quelconque des revendications 1 à 7, comprenant en outre un manchon disposé au moins partiellement circonférentiellement autour d'un élément au moins parmi le transducteur à ultrasons et l'élément allongé (22).

9. Manipulateur utérin (20) selon l'une quelconque des revendications 1 à 6, dans lequel le transducteur à ultrasons est déplaçable axialement le long de l'élément allongé (22).

10. Manipulateur utérin (20) selon la revendication 9, comprenant en outre un manchon disposé au moins partiellement circonférentiellement autour d'un élément au moins parmi le transducteur à ultrasons et l'élément allongé (22).

11. Manipulateur utérin (20) selon l'une quelconque des revendications 1 à 6, dans lequel le transducteur à ultrasons est déplaçable dans une direction radiale en rapprochement et en éloignement de la ligne centrale.

12. Manipulateur utérin (20) selon la revendication 11, dans lequel le transducteur à ultrasons est attaché en pivotement sur un élément parmi l'élément allongé et un manchon disposé au moins partiellement circonférentiellement autour de l'élément allongé.

13. Manipulateur utérin (20) selon l'une quelconque des revendications 1 à 6, dans lequel le transducteur à ultrasons est attaché de manière fixe à un élément parmi l'élément allongé (22) et le plot (38) qui est attaché de manière fixe sur l'élément allongé.

14. Manipulateur utérin (20) selon la revendication 7, dans lequel le transducteur à ultrasons est déplaçable axialement le long de l'élément allongé, et dans lequel le transducteur à ultrasons est déplaçable dans une direction radiale en rapprochement et en éloignement de la ligne centrale.

15. Manipulateur utérin (20) selon la revendication 14, dans lequel le transducteur à ultrasons est susceptible d'être fixé sélectivement sur l'élément allongé.

16. Manipulateur utérin (20) selon l'une quelconque des revendications 1 à 6, comprenant en outre une coupelle cervicale, le transducteur à ultrasons étant attaché à la coupelle cervicale, la coupelle cervicale étant au moins une coupelle parmi une coupelle en rotation autour de l'élément allongé et une coupelle en déplacement axial le long de l'élément allongé.
